# EUROPÄISCHE PATENTSCHRIFT

(11) **EP 0 912 497 B1**
(45) Veröffentlichungstag und Bekanntmachung des Hinweises auf die Patenterteilung: **02.02.2005**
(21) Anmeldenummer: 96930969.9
(22) Anmeldetag: 28.08.1996
(51) Int. Cl.: C07C 233/36, C07C 231/08, C11D 1/52

(54) **VERFAHREN ZUR HERSTELLUNG VON DI- ODER OLIGOAMIDALKOXYLATEN**
PROCESS FOR PREPARING DI- OR OLIGOAMIDE ALKOXYLATES
PROCEDE DE PREPARATION D'ALCOXYLATES DE DIAMIDES OU D'OLIGOAMIDES

(30) Priorität: 28.06.1996 DE 19625937
(43) Veröffentlichungstag der Anmeldung: 06.05.1999
(73) Patentinhaber: SASOL Germany GmbH, 22297 Hamburg (DE)
(72) Erfinder: JACOBS, Ulrike, D-45721 Haltern (DE); KALTWASSER, Uwe, D-45770 Marl (DE); KWETKAT, Klaus, D-44534 Lünen (DE); LEHMANN, Ernst-Jürgen, D-48249 Dülmen (DE)
(74) Vertreter: Schupfner, Gerhard D., Dr. Dipl.-Chem.
(86) Internationale Anmeldenummer: PCT/EP1996/003776
(87) Internationale Veröffentlichungsnummer: WO 1998/000392

(56) Entgegenhaltungen:
- EP-A- 0 038 862
- DE-A- 4 440 328
- GB-A- 835 566
- GB-A- 2 203 177

## Beschreibung

Für die Herstellung von Carbonsäuredi- oder -oligoamidalkoxylaten, wie sie in WO 95/19955 beschrieben werden, oder von Carbonsäuredi- oder -oligoamidpolyalkylenethersulfaten, wie sie in DE 44 40 328 beschrieben werden, ist die Bereitstellung eines möglichst reinen und an farbigen Verunreinigungen möglichst armen Rohstoffes mit der geforderten Grundstruktur sehr wichtig für die Eigenschaften und die Effizienz der daraus hergestellten Tenside bzw. Formulierungen.

In EP-A-0 038 862 wird beschrieben, wie ein effektives Weichspülmittel für Textilien gewonnen wird, wenn man Diethylentriamin oder ein höheres Homologes mit zwei Molen Fettsäure umsetzt und das entstehende Produkt am Aminstickstoff monoethoxyliert und zumindest teilweise mit einer niederen organischen Säure und/oder Schwefeldioxid neutralisiert.

In WO 95/19955 wird in einem Beispiel auf die Herstellung der detailliert beschriebenen Struktur eingegangen, wobei diese Beschreibung analog auch für andere beanspruchte Strukturen gelten soll. Hier soll in einem Dreischrittverfahren das verwendete Amin zunächst mit einem Mol Ethylenoxid pro primärer oder sekundärer Aminogruppe im Molelkül umgesetzt werden, anschließend die sekundären α- und ω-Ammogruppen mit Carbonsäuremethylester acyliert werden, um dann in einem dritten Schritt mit den restlichen für das Endprodukt notwendigen Alkoxydeinheiten umgesetzt zu werden.

Dieses Verfahren führt jedoch, wie dem Fachmann bekannt ist, zu einem Gemisch aller theoretisch möglichen Produkte, so daß die der beanspruchten Struktur entsprechenden Geminipolyetherfettsäureamide nur in Konzentrationen ≤ 50 mol % im Produktgemisch vorhanden sein können, dafür aber auch Fettsäureester von Aminalkoxylaten in größeren Mengen im Produkt enthalten sein müssen. Folglich wird bei diesem Verfahren zwar das Handling der oft hochschmelzenden Di- und Oligoamide erleichtert, doch geht das sehr drastisch zu Lasten der Produktqualität.

Das in DE 44 40 328 beschriebene Verfahren sieht den Einsatz von Di- oder Oligoamiden vor, die durch die Umsetzung von Aminen mit freien Carbonsäuren oder Carbonsäuremethylestern hergestellt worden sind. Damit ergibt sich besonders bei der Verwendung von Alkylendiaminen, aber auch bei anderen Spacereinheiten - hier sind die Sauerstoff enthaltenden Verbindungen obendrein nur bedingt temperaturbelastbar - das Problem, die Diamide nach der Herstellung wieder aufzuschmelzen, was sich nicht günstig auf Qualität und Farbe der Produkte auswirkt.

Das für konventionelle Fettsäurepolyether- und -polyethersulfatamide verwendete Verfahren, Mono- oder Diethanolamin zur Amidierung zu benutzen, ist in den oben beschriebenen Fällen nicht möglich, da die Amine zur Verknüpfung zweier Carbunsäureketten eingesetzt werden.

Es wurde nun überraschend gefunden, daß die beschriebenen Schwierigkeiten und Qualitätseinbußen vermieden werden, wenn die Diamide direkt nach der Herstellung ohne zwischenzeitliche Aufarbeitung oder Isolierung als Schmelze zur Alkoxylierung gelangen, wobei der für die Amidierung eingesetzte Katalysator auch für die Alkoxylierung weiterverwendet werden kann, oder daß vor der Alkoxylierung - einer Umsetzung mit Ethylen- und / oder Propylenoxid und bei Bedarf im Anschluß daran mit Butylenoxid - nachdosiert oder ein anderer Katalysator eingesetzt werden kann.

Gegenstand der Erfindung ist also ein Verfahren zur Herstellung von Di- oder Oligoamidalkoxylaten gemäß Anspruch 1.

Bei Verwendung freier Carbonsäuren kann auf den Katalysator verzichtet werden. In diesem Falle wird nach Beendigung der Amidierung der Alkoxylierungskatalysator zudosiert.

Die Reaktionen können als Eintopfreaktion oder alternativ in zwei verschiedenen Reaktoren durchgeführt werden. Die Reaktoren sollten dann zweckmäßigerweise räumlich nicht weit voneinander entfernt sein, doch ist auch eine größere räumliche Entfernung bei Verwendung beheizter Leitungen kein grundsätzliches Hindernis für das erfindungsgemäße Verfahren.

Das Verfahren ist dadurch gekennzeichnet, daß Carbonsäuren oder Carbonsäurealkylester, vorzugsweise Carbonsäuremethylester, mit 4 bis 24, vorzugsweise 8 bis 18, Kohlenstoffatomen in der zugrundeliegenden Carbonsäure und deren Mischungen untereinander vorgelegt werden, und das Di- Oligo- oder Polyamin anschließend zugemischt wird. Die Kohlenstoffkette der Carbonsäuren oder des Carbonsäuremethylesters kann verzweigt oder unverzweigt, cyclisch oder acyclisch, aliphatisch oder aromatisch sein. Als Di-, Oligo- oder Polyamin kommen für das erfindungsgemäße Verfahren alle sowohl in DE 44 40 328 als auch in WO 95/19955 beschriebenen Amine in Frage. Die einzige Maßgabe für die Mischung ist, daß die Komponenten in flüssigem Zustand in einen geeigneten Reaktor gefüllt werden. Alle Schritte erfolgen mit Stickstoffabdeckung. Für eine hohe Farbqualität ist der weitgehende Ausschluß von Luftsauerstoff notwendig. Als letztes wird der Katalysator, wenn notwendig, zugesetzt, da er sonst, z. B. bei der Verwendung von Carbonsäuremethylestern, zur Verseifung des Rohstoffes führt. Als Katalysator kommen alle für die Amidierung geeigneten Katalysatoren in Frage, wie z. B. Kalium- oder Natriummethanolat, Kalium- oder Natriumethanolat, Kaliumhydroxid, Natriumhydroxid, Zinnoxalat, elementares Zinn, Zinkoxid und Kalium-tert.-butanolat, besonders bevorzugt sind Natrium- und Kaliumalkoholate und -hydroxide. Der Katalysator wird in Konzentrationen von 0 bis 5 Gew.-%, bevorzugt 0 bis 3 und besonders bevorzugt 0 bis 1 Gew.-%, bezogen auf den Gesamtansatz (für die Amidierung) eingesetzt.

Nach Mischung der Reaktanden wird die Innentemperatur des Reaktors auf mindestens 2 bis 5 °C über der Erstarrungstemperatur des Produktdiamides und maximal auf 240 °C, bevorzugt auf 80 bis 200 °C und besonders bevorzugt auf 100 bis 190 °C, erhöht. Alternativ wird sofort mit der Entfernung von Wasser oder Methanol, je nach Einsatzstoff, begonnen oder bis zur Einstellung einer konstanten Sumpf- und Kopftemperatur, also der Einstellung des Reaktionsgleichgewichtes, gewartet. Die dazu notwendigen Temperaturen hängen von dem konkreten Rohstoffgemisch ab und liegen im obengenannten Bereich. Die notwendigen Zeiten liegen im Bereich von 10 bis 120, bevorzugt 15 bis 60 und besonders bevorzugt 15 bis 40 Minuten.

Die Methanol- bzw. Wasserentfernung findet entweder unter Atmosphärendruck unter Verwendung eines leichten in das Reaktionsgemisch eingeleiteten Stickstoffstromes oder unter Vakuum statt. Einbauten, die zu einer Trennwirkung von 0,1 bis 20, vorzugsweise 0,5 bis 10 theoretischen Trennböden führen, sind zur Steigerung der Selektivität zweckmäßig.

Nach Entfernung des Methanols bzw. des Wassers wird die Temperatur auf 130 bis 180 °C eingestellt. An dieser Stelle kann Katalysator zugesetzt werden. Die verwendbaren Katalysatoren sind alle für Alkoxylierungen in Frage kommenden Katalysatoren einschließlich der zu engen Homologenverteilungen führenden. Als Beispiele seien Natrium- und Kaliumalkoxylate und -hydroxide, Schichtsilikate und Hydrotalcit genannt. Bevorzugt werden Natrium- und Kaliumalkoxylate und -hydroxide.

Die Alkoxylierung wird mit der notwendigen Menge Alkylenoxid entsprechend dem Stand der Technik bei 2 bis 6 bar Gesamtdruck und 130 bis 180 °C, durchgeführt. Im Anschluß an die Nachreaktionszeit wird das Produkt bei 40 bis 160 °C, bevorzugt 45 bis 120 °C, abgelassen. Soll das Alkoxylat als Tensid eingesetzt werden, muß der Katalysator, z. B. mit Carbonsäuren mit niedrigem Molekulargewicht, wie Milchsäure, neutralisiert werden. Bei Verwendung heterogener Katalysatoren schließt sich eine Filtration an. Wird das Alkoxylat als Endprodukt verwendet, kann bei Bedarf, z. B. mit Wasserstoffperoxid, gebleicht werden.

Für die anschließende Sulfierung, wie sie z. B. in DE 44 40 328 beschrieben wird, kann wahlweise auf die Neutralisierung verzichtet werden und die Sulfierung mit einem SO₃ / Luft - Gemisch oder Oleum oder Chlorsulfonsäure oder Amidosulfonsäure, bevorzugt jedoch mit einem SO₃ / Luft - Gemisch, direkt im Anschluß durchgeführt werden. Im Anschluß daran erfolgt die Neutralisation mit entsprechenden Äquivalenten an Alkali- oder Erdalkalihydroxiden oder mit entsprechenden Äquivalenten von Mono-, Di- und Trialkanolaminen. Dank der schonenden Herstellung der Vorstufen können auch nach diesem Schritt Produkte hoher Farbqualität der gewünschten Struktur hergestellt werden. Je nach Bedarf kann sich an diesen Schritt eine Bleichung, z. B. mit Wasserstoffperoxid, anschließen.

Das erfindungsgemäße Verfahren zeichnet sich dadurch aus, daß es erlaubt, die in DE 44 40 328 bzw. WO 95/19955 beschriebenen Strukturen in hoher Reinheit mit mehr als 70 mol-%, bevorzugt mehr als 80 mol-% und besonders bevorzugt mehr als 85 mol% herzustellen und damit zu effizienteren, also auch kostengünstigeren Tensiden respektive Formulierungen zu kommen.

Die folgenden Beispiele sollen das erfindungsgemäße Verfahren erläutern jedoch nicht darauf einschränken.

### Beispiel 1:

| | | |
|---|---|---|
| 203,9 kg | C8/C10-Fettsäuremethylester | 1,20 kmol |
| 36,1 kg | Ethylendiamin | 0,60 kmol |
| 2,4 kg | K-Methylat-Pulver | |
| 264 kg | Ethylenoxid | 6,00 kmol |

In einem geeigneten Reaktor mit 1 000 1 Volumen wurden der Fettsäuremethylester und das Amin vereinigt und dann der Katalysator zugefügt. Die Mischung wurde zum Rückfluß erhitzt, der bei einer Sumpftemperatur von etwa 115 °C einsetzte. Bei Eintritt des Reaktionsgleichgewichtes nach etwa 45 Minuten stellte sich eine Sumpftemperatur von ca. 102 °C und eine Kopftemperatur von 65 °C ein. Das gebildete Methanol wurde abdestilliert und dabei die Sumpftemperatur auf 175 °C gesteigert.

Nach Umschaltung der Apparatur auf Ethoxylierungsbetrieb wurde unmittelbar mit der Reaktion fortgefahren. Bei einer Reaktionstemperatur von 165 °C wurden 0,5 bar Stickstoff aufgepreßt und mit Ethylenoxid (E0) auf 3,0 bar ergänzt, wobei dieser Druck durch laufende Zufuhr des E0 aufrechterhalten wurde. Nach etwa 1,5 h war die vorgesehene E0-Menge aufgenommen. Nach einer Nachreaktionszeit von 1 h, bei der der Reaktordruck auf ca. 1 - 1,5 bar abfiel, wurden mit Stickstoff enthaltene Reste an E0 ausgeblasen und anschließend das Reaktionsprodukt unter Stickstoffspülung abgelassen. Das Produkt ist hell, Iod-Farbzahl von 27, und entspricht laut ¹³C NMR der geforderten Struktur.

### Beispiel 2

| | | |
|---|---|---|
| 203,9 kg | C8/C10-Fettsäuremethylester | 1,20 kmol |
| 44,5 kg | 1,2-Diaminopropan | 0,60 kmol |
| 2,4 kg | K-Methylat-Pulver | |
| 264 kg | Ethylenoxid | 6,00 kmol |

In einem geeigneten Reaktor mit 1 000 l Volumen wurden der Fettsäuremethylester und das Amin vereinigt und dann der Katalysator zugefügt. Die Mischung wurde zum Rückfluß erhitzt, der bei einer Sumpftemperatur von etwa 120 °C einsetzte. Bei Eintritt des Reaktionsgleichgewichtes nach etwa 40 Minuten stellte sich eine Sumpftemperatur von ca. 106 °C und eine Kopftemperatur von 70 °C ein. Das gebildete Methanol wurde abdestilliert und dabei die Sumpftemperatur auf 180°C gesteigert.

Nach Umschaltung der Apparatur auf Ethoxylierungsbetrieb wurde unmittelbar mit der Reaktion fortgefahren. Bei einer Reaktionstemperatur von 165 °C wurden 0,5 bar Stickstoff aufgepreßt und mit E0 auf 3,0 bar ergänzt, wobei dieser Druck durch laufende Zufuhr des E0 aufrechterhalten wurde. Nach etwa 1,5 h war die vorgesehene E0-Menge aufgenommen. Nach einer Nachreaktionszeit von 1 h, bei der der Reaktordruck auf ca. 1 - 1,5 bar abfiel, wurden mit Stickstoff enthaltene Reste an E0 ausgeblasen und anschließend das Reaktionsprodukt unter Stickstoffspülung abgelassen. Das Produkt ist hell, Iod-Farbzahl von 28, und entspricht laut ¹³C NMR der geforderten Struktur.

## Patentansprüche

1. Verfahren zur Herstellung von Di- oder Oligoamidalkoxylaten, **dadurch gekennzeichnet, dass**
- man Carbonsäuren oder Carbonsäurealkylester mit Di-, Oligo- oder Polyaminen umsetzt und
- das Reaktionsprodukt ohne zwischenzeitliche Isolierung oder Aufarbeitung als Schmelze zu Di- oder Oligoamidalkoxylaten unter Einsatz eines Alkoxylierungskatalysators alkoxyliert, wobei die Alkoxylierung bei 130 bis 180°C durchgeführt wird und die Di- oder Oligoamidalkoxylate am Stickstoffatom der Amidgruppe alkoxyliert sind.

2. Verfahren nach Anspruch 1, **dadurch gekennzeichnet, daß** Carbonsäuren oder Carbonsäurealkylester mit 4 bis 24, vorzugsweise 8 bis 18, Kohlenstoffatomen im Kohlenwasserstoffrest der zugrundeliegenden Carbonsäure eingesetzt werden.

3. Verfahren nach mindestens einem der vorhergehenden Ansprüche, **dadurch gekennzeichnet, dass** der für die Amidierung eingesetzte Katalysator auch für die Alkoxylierung verwendet wird.

4. Verfahren nach mindestens einem der vorhergehenden Ansprüche, **dadurch gekennzeichnet, daß** bei Einsatz von Carbonsäuren für die Amidierung kein Katalysator zugesetzt wird.

5. Verfahren nach mindestens einem der vorhergehenden Ansprüche, **dadurch gekennzeichnet, daß** für die Alkoxylierung ein von dem für die Amidierung verwendeten Katalysator, verschiedener Katalysator eingesetzt bzw. nachdosiert wird.

6. Verfahren nach mindestens einem der vorhergehenden Ansprüche, **dadurch gekennzeichnet, daß** die Komponenten in flüssigem Zustand in den Reaktor eingefüllt werden.

7. Verfahren nach mindestens einem der vorhergehenden Ansprüche, **dadurch gekennzeichnet, daß** die Amidierung und die Alkoxylierung im Eintopfverfahren durchgeführt werden.

8. Verfahren nach mindestens einem der vorhergehenden Ansprüche, **dadurch gekennzeichnet, dass** die Innentemperatur des Reaktors auf mindestens 2 °C über der Erstarrungstemperatur des Produktamides und maximal auf 240 °C, vorzugsweise auf 80 bis 200 °C, erhöht wird.

9. Verfahren nach mindestens einem der vorhergehenden Ansprüche, **dadurch gekennzeichnet, daß** die Produkte einer Bleichung unterzogen werden.

10. Verfahren nach mindestens einem der vorhergehenden Ansprüche, **dadurch gekennzeichnet, daß** die Verbindungen in einer Reinheit von mehr als 70 Mol-% erhalten werden.

11. Verfahren nach mindestens einem der vorhergehenden Ansprüche, **dadurch gekennzeichnet, daß** die Verbindungen in einer Reinheit von mehr als 85 Mol-% erhalten werden.

12. Verfahren nach mindestens einem der vorhergehenden Ansprüche, **dadurch gekennzeichnet, daß** man Diamine zu Diamiden umsetzt und alkoxyliert.

13. Verfahren nach mindestens einem der vorstehenden Ansprüche, **dadurch gekennzeichnet, dass** der Alkoxylierungskatalysators ausgewählt ist aus der Gruppe Natriumund Kaliumalkoxylate, Kaliumhydroxid, Schichtsilikate und Hydrotalcit.

## Claims

1. A process for preparing di- or oligoamide alkoxylates,
**characterized in that**
- carboxylic acids or carboxylic acid alkyl esters are reacted with di-, oligo-, or polyamines and
- the reaction product is alkoxylated to di-, or oligoamide alkoxylates as a melt with the use of an alkoxylation catalyst without any intermediate isolation or treatment, wherein the alkoxylation is conducted at 130 to 180 °C and the di-, or oligoamide alkoxylates are alkoxylated at the nitrogen atom of the amide group.

2. A process according to claim 1,
**characterized in that**
carboxylic acids or carboxylic acid alkyl esters having 4 to 24, preferably 8 to 18, carbon
atoms in the hydrocarbon residue of the underlying carboxylic acid are used.

3. A process according to at least one of the preceding claims,
**characterized in that**
the catalyst used for the amidation is also employed for the alkoxylation.

4. A process according to at least one of the preceding claims,
**characterized in that**
no catalyst is added for the amidation in case carboxylic acids are used.

5. A process according to at least one of the
preceding claims, **characterized in that** a catalyst which is different from the one used for the amidation is employed or added for the alkoxylation.

6. A process according to at least one of the
preceding claims, **characterized in that** the components are charged to the reactor in the liquid state.

7. A process according to at least one of the preceding claims,
**characterized in that**
amidation and alkoxylation are performed as a one-pot process.

8. A process according to at least one of the preceding claims,
**characterized in that**
the internal reactor temperature is increased to at least 2 °C above the solidification temperature of the amide product and to max.
240°C, preferably 80 to 200 °C.

9. A process according to at least one of the preceding claims,
**characterized in that**
the products are subjected to bleaching.

10. A process according to at least one of the preceding claims,
**characterized in that**
the compounds are obtained in purities of greater than 70 mol-%.

11. A process according to at least one of the preceding claims,
**characterized in that** the compounds are
obtained in purities of greater than 85 mol-%.

12. A process according to at least one of the preceding claims,
**characterized in that**
diamines are converted to diamides and alkoxylated.

13. A process according to at least one of the preceding claims,
**characterized in that** that the alkoxylation catalyst is selected from the group consisting of sodium and potassium alkoxylate, potassium hydroxide, lattice layer silicates and hydrotalcites.

## Revendications

1. Procédé pour la préparation de di- ou oligoamidalcoxylates, **caractérisé en ce que** l'on
- met à réagir des acides carboxyliques ou des esters alkyliques d'acides carboxyliques avec des di-, oligo- ou polyamines et
- réalise l'alcoxylation du produit de réaction sans séparation intermédiaire ni transformation à l'état fondu en di- ou oligoamidalcoxylates en utilisant un catalyseur d'alcoxylation, l'alcoxylation étant réalisée entre 130 et 180 °C et les di- ou oligoamidalcoxylates étant alcoxylés sur l'atome d'azote du groupe amide.

2. Procédé selon la revendication 1, **caractérisé en ce que** l'on utilise des acides carboxyliques ou des esters alkyliques d'acides carboxyliques avec de 4 à 24, de préférence de 8 à 18, atomes de carbone dans le radical hydrocarboné de l'acide carboxylique de base.

3. Procédé selon au moins l'une quelconque des revendications précédentes, **caractérisé en ce que** le catalyseur utilisé pour l'amidation est également utilisé pour l'alcoxylation.

4. Procédé selon au moins l'une quelconque des revendications précédentes, **caractérisé en ce que**, lors de l'utilisation d'acides carboxyliques pour l'amidation on n'ajoute pas de catalyseur.

5. Procédé selon au moins l'une quelconque des revendications précédentes, **caractérisé en ce que** l'on utilise ou l'on dose ultérieurement pour l'alcoxylation un catalyseur différent du catalyseur utilisé pour l'amidation.

6. Procédé selon au moins l'une quelconque des revendications précédentes, **caractérisé en ce que** l'on verse les composants dans le réacteur à l'état liquide.

7. Procédé selon au moins l'une quelconque des revendications précédentes, **caractérisé en ce que** l'on réalise l'amidation et l'alcoxylation dans un procédé en cuve unique.

8. Procédé selon au moins l'une quelconque des revendications précédentes, **caractérisé en ce que** l'on augmente la température interne du réacteur à au moins 2 °C au-dessus de la température de solidification de l'amide du produit et au maximum à 240 °C, de préférence à 80 jusqu'à 200 °C.

9. Procédé selon au moins l'une quelconque des revendications précédentes, **caractérisé en ce que** les produits sont soumis à un blanchiment.

10. Procédé selon au moins l'une quelconque des revendications précédentes, **caractérisé en ce que** l'on obtient les composés avec une pureté de plus de 70 % en moles.

11. Procédé selon au moins l'une quelconque des revendications précédentes, **caractérisé en ce que** l'on obtient les composés avec une pureté de plus de 85 % en moles.

12. Procédé selon au moins l'une quelconque des revendications précédentes, **caractérisé en ce que** l'on transforme et l'on alcoxyle des diamines en diamides.

13. Procédé selon au moins l'une quelconque des revendications précédentes, **caractérisé en ce que** le catalyseur d'alcoxylation est choisi dans le groupe des alcoxylates de sodium et de potassium, de l'hydroxyde de potassium, des silicates lamellaires et de l'hydrotalcite.
